# EUROPÄISCHE PATENTSCHRIFT

(11) **EP 1 904 973 B1**
(45) Veröffentlichungstag und Bekanntmachung des Hinweises auf die Patenterteilung: **02.09.2009**
(21) Anmeldenummer: 07725926.5
(22) Anmeldetag: 08.06.2007
(51) Int. Cl.: G06T 7/00

(54) **VERFAHREN, VORRICHTUNG UND COMPUTERPROGRAMMPRODUKT ZUM AUSWERTEN VON BILDERN EINER KAVITÄT**
METHOD, DEVICE AND COMPUTER PROGRAMME FOR EVALUATING IMAGES OF A CAVITY
PROCÉDÉ, DISPOSITIF ET PRODUIT DE PROGRAMME INFORMATIQUE DESTINÉS À L'ÉVALUATION D'IMAGES D'UNE CAVITÉ

(30) Priorität: 08.06.2006 DE 102006026695
(43) Veröffentlichungstag der Anmeldung: 02.04.2008
(73) Patentinhaber: TomTec Imaging Systems GmbH, 85716 Unterschleissheim (DE)
(72) Erfinder: SCHUMMERS, Georg, 80935 München (DE)
(74) Vertreter: Schinkel, Reta
(86) Internationale Anmeldenummer: PCT/EP2007/005094
(87) Internationale Veröffentlichungsnummer: WO 2007/141038

(56) Entgegenhaltungen:
- EP-A- 0 961 135
- WO-A-01/16886
- US-A- 5 800 355
- US-A- 5 970 182
- US-A1- 2004 087 853
- US-B1- 6 248 070
- KAPETANAKIS S ET AL: "Real-time three-dimensional echocardiography: a novel technique to quantify global left ventricular mechanical dyssynchrony." CIRCULATION 16 AUG 2005, Bd. 112, Nr. 7, 16. August 2005 (2005-08-16), Seiten 992-1000, XP002451647 ISSN: 1524-4539 in der Anmeldung erwähnt
- BAX JEROEN J ET AL: "Cardiac resynchronization therapy: Part 1--issues before device implantation." JOURNAL OF THE AMERICAN COLLEGE OF CARDIOLOGY 20 DEC 2005, Bd. 46, Nr. 12, 20. Dezember 2005 (2005-12-20), Seiten 2153-2167, XP002451648 ISSN: 1558-3597 in der Anmeldung erwähnt

## Beschreibung

Die Erfindung betrifft ein Verfahren zum Auswerten von Bildern einer Kavität im menschlichen oder tierischen Körper, die mit medizinischen Bildgebungsverfahren aufgenommen wurden, insbesondere mit Realtime-3D-Ultraschall, Röntgenverfahren, Computertomographie (CT), Magnetresonanztomographie (MRT), einer elektrophysiologischen Herzkatheteruntersuchung, oder nuklearmedizinischen Verfahren wie Positronen-Emissionstomographie (PET) oder (Single Proton Emission Computed Tomography (SPECT). Insbesondere handelt es sich dabei um dynamische - also in einer Zeitreihe hintereinander aufgenommene - Bilder z.B. des Herzens. Die Auswertung wird bevorzugt nicht direkt an den Bildern vorgenommen, sondern an Datensätzen mit funktionellen Werten, welche aus den Bildern gewonnen bzw. berechnet wurden und z. B. die Herzkammerwand-Aktivität einer Herzkammer beschreiben.

Für die Bewertung von Funktionalstörungen werden heutzutage verschiedene Bildgebungsmodalitäten bzw. -verfahren verwendet, welche die Akquisition von dynamischen dreidimensionalen Bildern erlauben, zum Beispiel Ultraschall, Magnetresonanztomographie (MRT), Positronen-Emissionstomographie (PET) oder (Single Proton Emission Computed Tomography (SPECT). Mit "dynamisch" ist gemeint, dass eine Zeitreihe von Bildern akquiriert wird, welche die Visualisierung von z. B. der Herzbewegung erlaubt. Besonders geeignet sind hierfür Realtime-3D-Ultraschallsysteme, mit denen Echtzeitaufnahmen in 3D aufgenommen werden können.

Zur Funktionalauswertung wird dann z. B. die Wand einer Kavität wie der Herzkammer betrachtet und z. B. deren Bewegung verfolgt. Eine Störung beispielsweise der Herzmuskel-Aktivität lässt sich z. B. daran erkennen, dass die Herzkammerwand nicht überall gleichzeitig und nicht überall gleich stark kontrahiert. Dies kann dadurch ermittelt werden, dass die Konturen der Kammer (zum Beispiel des linken oder rechten Ventrikels oder einer Vorkammer) automatisch detektiert und betrachtet werden. Die Innenseite der Kavität kann dann als eine Art Gitternetzstruktur, wie sie beispielsweise in der EP 0 961 135 der Anmelderin beschrieben wurde, dargestellt werden, deren Oberfläche zum Beispiel durch Dreiecke angenähert wird (im Weiteren "Beutel" genannt). Der Beutel wird dann in Abschnitte eingeteilt, und die Bewegung der einzelnen Abschnitte wird verfolgt, um zum Beispiel die Kontraktionszeit und die jeweiligen Verzögerungszeiten gegenüber dem Abschnitt der frühesten Kontraktion zu ermitteln. Die Kavität (z. B. des Herzens) kann auch in Volumenabschnitte eingeteilt werden, deren Volumenänderung z. B. über einen Herzschlag betrachtet wird. Die Ergebnisse solcher Auswertungen werden oft in Form eines so genannten "Polarplots" dargestellt, welcher z. B. das Herz wie auf eine flache Landkarte ausgebreitet darstellt, wobei jedem Abschnitt der Herzkammerwand ein Sektor im Polarplot zugewiesen ist. Ein Polarplot stellt also praktisch eine flache Landkarte z. B. einer Herzkammer dar, auf der bestimmte funktionelle Werte wie Kontraktionszeit oder maximale Volumenänderung farbcodiert aufgetragen sind.

Derartige Auswertungen werden zum Beispiel in der Druckschrift US 2004/0087853 A1 gezeigt, und mit den Softwareprogrammen TomTec Left Ventricular-Analysis, TomTec Right Ventricular-Analysis und 4D Left Ventricular-Analysis Cardiac Resynchronization Therapy sowie z. B. mit PET/SPECT-Softwares durchgeführt. Die in diesen Programmen verwendeten Auswertungsmethoden zur Gewinnung von Datensätzen mit funktionellen Werten sind z.B. in den folgenden Artikeln beschrieben:
Kapetanakis, Monaghan:L "Real-Time Three-Dimensional Echocardiography - A Novel Technique to Quantify Global Left Ventricular Mechanical Dyssynchrony", Circulation. 2005;112:992-1000.
Bax JJ, Abraham T, Barold SS, Breithardt OA, , Mark DB, Monaghan MJ, Nihoyannopoulo: "Cardiac resynchronization therapy: Part 1--issues before device implantation", J Am Coll Cardiol. 2005 Dec 20;46(12):2168-82.
Monaghan MJ: "Role of real time 3D echocardiography in evaluating the left ventricle", Heart. 2006 Jan;92(1):131-6.
Lipiec P, Piewka M, Kasprzak JD: "Automated quantification of left-ventricular volumes and function: a novel clinical tool?" Congress of the European Society of Cardiology 2003

Die Ergebnisse solcher Auswertungen werden dann als mehrdimensionaler Datensatz mit funktionellen Werten ausgegeben, zum Beispiel in Form eines Beutels, der aus Punkten, Dreiecken oder dergleichen aufgebaut sein kann und darüber hinaus auch funktionelle Werte enthalten kann, als Polarplot oder als Wertetabelle.

Der Kardiologe steht oft vor der Aufgabe, verschiedene funktionelle Auswertungen der Herzkammern miteinander zu vergleichen, zum Beispiel bei einer Stressechountersuchung, bei der funktionell gestörte Areale im Myokard des linken Ventrikels in Ruhe sowie bei Belastung des Patienten bestimmt werden. Bislang geschieht ein Vergleich zwischen Ruhe- und Belastungszustand in der Routine immer mittels subjektiver Bewertung durch den Arzt. Ferner müssen gelegentlich mit verschiedenen Bildgebungsmodalitäten gewonnene Informationen miteinander verglichen werden, z.B. ein durch PET oder SPECT gewonnenes Perfusionsbild des Herzens mit einem dynamischen Datensatz, welcher die Aktivität der Herzkammerwand zeigt.

Ein standardisierter, qualitativer Vergleich existiert lediglich auf der Basis von 2D-Schnitten des Herzens. Dieser Standard wurde vom American Congress of Cardiology (ACC) sowie von der American Heart Association (AHA) entwickelt. Dieses Verfahren bietet allerdings nur eine sehr begrenzte räumliche Abtastung des linken Ventrikels, da nur einige wenige bestimmte 2D Schnitte (drei Langachsen-Schnitte und ein Kurzachsen-Schnitt) und nicht der gesamte Ventrikel vom Untersucher bewertet werden. Die Befundungen sind durch ihre Inter- und Intra-Observer-Variabilität nicht gut vergleichbar.

Im Stand der Technik sind daher keine Verfahren zum Auswerten von dynamischen Bildern z. B. des Herzens bekannt, die eine vollständig räumliche vergleichende Auswertung und somit einen objektiven Befund erlauben. Durch die unterschiedlichen Formate, in denen die funktionellen Auswertungen der Herzkammern bereitgestellt werden, besteht auch keine Möglichkeit, verschiedene Untersuchungsergebnisse auf einen Blick zu erfassen.

Die Erfindung hat sich daher die Aufgabe gestellt, ein Verfahren zum Auswerten von Bildern einer Kavität im menschlichen oder tierischen Körper bereitzustellen, welches die vorgenannten Nachteile überwindet.

Diese Aufgabe löst die Erfindung mit den Merkmalen des Anspruchs 1. Vorteilhafte Ausgestaltungen der Erfindung sind in den Unteransprüchen angegeben.

Die Erfindung zeichnet sich darin aus, dass mindestens zwei Datensätze mit funktionellen Werten in jeweils einem unterschiedlichen Datenformat bereitgestellt werden, insbesondere in Form einer Wertetabelle, einer Projektion auf eine 2D Ebene (z. B. eines Polarplots) oder einer Gitternetzstruktur (z. B. eines Beutel-Datensatzes). Die Datensätze mit funktionellen Werten sollen z.B. miteinander verglichen werden. Jeder Datensatz wird bevorzugt aus einem mit einem bestimmten Bildgebungsverfahren aufgenommenen Satz von Bildern der Kavität gewonnen bzw. berechnet. Die Erfindung zeichnet sich ferner dadurch aus, dass ein einheitliches Datenformat bereitgestellt, insbesondere von einem Benutzer ausgewählt wird, dass der/die nicht in diesem einheitlichen Datenformat bereitgestellten Datensätze mit funktionellen Werten in das einheitliche Datenformat umgewandelt werden; dass die dann im einheitlichen Datenformat vorliegenden Datensätze miteinander zur Erzeugung von zumindest einem Ergebnis-Datensatz mindestens einmal miteinander verrechnet (z.B. addiert oder subtrahiert) werden; und schließlich der Ergebnis-Datensatz dargestellt wird.

Vorzugsweise ist die Kavität eine Kammer des Herzens, z.B. der linke Ventrikel, der rechte Ventrikel, der linke oder rechte Vorhof, oder ein anderes Blutgefäß wie die Aorta. Die Erfindung kann jedoch auch auf andere Kavitäten wie den Darm, den Magen, einen Hirnventrikel, die Blase etc. angewendet werden.

Die Datensätze mit funktionellen Werten (im Folgenden auch "funktionelle Datensätze" genannt) wurden jeweils aus Bildern der Kavität gewonnen bzw. berechnet, die mit einem medizinischen Bildgebungsverfahren aufgenommen wurden. Der Ausdruck "Bilder der Kavität" ist hier sehr umfassend zu verstehen, es können hiermit auch Messdaten gemeint sein, die nach Akquisition nicht direkt in Bildform vorliegen, z.B. die Ergebnisse einer Abtastung der Herzkammerwand mit einer Elektrode, welche eine räumliche Verteilung des elektrischen Potentials liefert. Da diese Messdaten auch räumliche Informationen enthalten, sind sie im weitesten Sinn auch als "Bilder" zu verstehen. Aus den Bildern des Potentials kann ein funktioneller Datensatz gewonnen werden, welcher das elektrische Potential in den abgetasteten Abschnitten der Herzkammerwand wiedergibt.

Die "Bilder der Kavität" können ferner die Ergebnisse einer Perfusionsmessung sein, welche z.B. mit einem nuklearmedizinischen Verfahren wie PET oder SPECT durchgeführt wurde. Der daraus gewonnen funktionelle Datensatz enthält somit Durchblutungswerte für mehrere Abschnitte der Herzkammerwand. Da die räumliche Auflösung von PET und SPECT gering ist, sind die einzelnen Abschnitte der Herzkammerwand sehr groß, denen jeweils ein Perfusionswert zugeordnet wird.

Die "Bilder der Kavität" können auch zweidimensionale (2D), dreidimensionale (3D) oder vierdimensionale (4D) Bilder sein, die mit MRT, Röntgen, CT oder Ultraschall akquiriert wurden. Ein 4D Bild ist eine Reihe von zeitlich nacheinander aufgenommen 3D Bildern. Handelt es sich bei der Kavität um eine Herzkammer, so deckt ein 4D Bild vorzugsweise einen Herzzyklus ab.

Besonders bevorzugt sind die "Bilder der Kavität" 4D-Ultraschall Bilder. Aus diesen Bildern lassen sich zahlreiche verschiedene Datensätze mit funktionellen Werte ermitteln, insbesondere solche, welche mit der Bewegung der Herzkammerwand zusammenhängen, z.B. die maximale relative Auslenkung der Herzkammerwand, die zeitliche Verzögerung der maximalen Auslenkung der Herzkammerwand, die Volumenänderung eines Abschnitts der Herzkammer während eines Herzzyklus, die Wanddicke der Herzkammer, Strain, Strain Rate etc. Der Strain eines Muskels ist das Verhältnis der momentanen Länge zu der maximalen (entspannten) Länge des Muskels und kann aus der Veränderung der Herzkammerwanddicke beim Herzzyklus berechnet werden.

Somit können die Bilder der Kavität entweder statisch sein (beim Herzen z.B. EKG-getriggert aufgenommen), oder dynamisch d.h. zeitabhängig. Ebenso können auch die funktionellen Datensätze entweder Werte enthalten, die über einen Herzschlag gleich bleiben (z.B. die Volumenänderung während eines Herzzyklus') oder dynamische Werte, wie z.B. die momentane Bewegung der Herzkammerwand.

Aus dem oben gesagten ergibt sich, dass die verschiedenen Datensätze mit funktionellen Werten, die aus Bildern von unterschiedlichen Bildgebungsmodalitäten gewonnenen wurden, oder die auf unterschiedliche Weise aus den Bildern der Kavität ermittelt wurden, in völlig unterschiedlichen Datenformaten vorliegen können. Zwar enthalten die funktionellen Datensätze alle in der Regel räumliche Informationen zu den funktionellen Werten, diese haben jedoch unterschiedliche Koordinatensysteme und Auflösungen. Die Datensätze mit funktionellen Werten sind vorzugsweise mehrdimensional, insbesondere zwei- oder dreidimensional, können jedoch auch eindimensional sein.

Die zu vergleichenden funktionellen Werte können zum Beispiel die Bewegung der Herzkammerwand, die relative Auslenkung der Herzkammerwand, die zeitliche Verzögerung der maximalen Auslenkung der Herzkammerwand, die Volumenänderung eines Abschnitts der Herzkammer, Strain, Strain Rate, Perfusion, E-lektropotentiale, sowie deren mathematischen Ableitungen, jeweils für einen Abschnitt der Herzkammerwand, repräsentieren.

Die funktionellen Werte können sowohl Skalare als auch Vektoren sein, wobei eine Vektorgröße z.B. die Bewegung der Kammerwand (Bewegungsrichtung und maximale Geschwindigkeit) oder die elektrische Aktivierung der Kammerwand (Richtung und Größe eines Aktionspotentials) repräsentieren kann.

Die Erfindung ermöglicht daher einen beliebigen Vergleich bzw. eine Verrechnung von funktionellen Datensätzen, die in unterschiedlichen Datenformaten, z.B. in Form von Beuteldaten, Polarplots oder Wertetabellen vorliegen. Es wird hierbei ein einheitliches Datenformat verwendet, in das die anderen Datensätze umgewandelt werden. Das einheitliche Datenformat ist entweder vorab festgelegt oder wird von einem Benutzer für eine Analyse festgelegt. Das einheitliche Datenformat kann auch ein Format sein, in dem einer oder mehrere der funktionellen Datensätze bereits vorliegen. Besonders bevorzugt ist das einheitliche Datenformat ein Polarplot oder ein Beutel-Datensatz, wie er in EP 961 135 A beschrieben ist.

Bei der Umwandlung der funktionellen Datensätze in das einheitliche Datenformat werden insbesondere die verschiedenen Koordinatensysteme der Datensätze zueinander in Bezug gesetzt und einander umgerechnet, z.B. kartesische Koordinaten in Polarkoordinaten und umgekehrt. Dies wird ermöglicht durch das allen Datensätzen gemeinsame Bezugsystem der Kavität - i.d.R. werden alle Datensätze Werte enthalten, die räumlich den Wänden der Kavität zugeordnet sind, so dass die Koordinatensysteme der Datensätze auf diese Weise aufeinander abgebildet werden können. Sind in einem funktionellen Datensatz also z.B. Werte für das elektrische Potential der Herzkammerwand mit einer räumlichen Angabe (x; y; z) im kartesischen 3D System angegeben, so können hieraus Polarkoordinaten für einen Polarplot berechnet werden, sofern - im Fall z.B. des linken Ventrikels - die Position des Apex und die des Mitralannulus (der oberen Begrenzung des linken Ventrikels) im 3D System bekannt ist.

Vorzugsweise werden bei der Umwandlung auch die verschiedenen räumlichen Auflösungen der funktionellen Datensätze berücksichtigt werden. Z.B. werden die Datensätze mit geringer räumlicher Auflösung überabgetastet oder interpoliert, um eine höhere räumliche Auflösung zu erreichen. Die Datensätze mit hoher räumlicher Auflösung werden z.B. unterabgetastet oder es wird über mehrere Datenpunkte gemittelt, um eine niedrigere räumliche Auflösung zu erreichen. Hierzu können bekannte Interpolations- und Approximationsverfahren verwendet werden.

Nach der Umwandlung in das einheitliche Datenformat werden die funktionellen Datensätze miteinander verrechnet, um sie auszuwerten bzw. miteinander zu vergleichen. Die geschieht vorzugsweise, indem jeder Datenpunkt eines ersten funktionellen Datensatzes mit dem seiner räumlichen Position entsprechenden Datenpunkt des zweiten bzw. der weiteren Datensätze verrechnet wird, z.B. durch Subtraktion voneinander, Addition, Multiplikation, Division oder Kombinationen davon. Möglich ist auch eine Ableitung und Integration z.B. über mehrere Datensätze, die aus zeitlich aufeinander folgenden Bildern gewonnen wurden.

Die Verrechnung kann auch Modell-basiert erfolgen. D. h., dass bei der Verrechnung mathematische Modelle der Kavität berücksichtigt werden können, z. B. der Verlauf von Erregungsleitungen in der Herzkammerwand, um die Eingangsdaten miteinander zu verrechnen. Der Verlauf der Erregungsleitungen in der Herzwand z.B. kann gemessen oder aus anderen Daten berechnet werden. Bei der Verrechnung von zwei funktionellen Datensätzen werden die Erregungsleitungen dann durch eine räumliche Abbildungsfunktion "aufeinander gelegt". D.h., es werden jeweils die räumlichen Abschnitte miteinander verglichen, die die gleiche relative Lage zu den Erregungsleitungen aufweisen.

Bei der Modell-basierten Verrechnung werden also nicht nur Punkte miteinander verrechnet, die die gleiche Lage im Raum haben, sondern zwischen denen z. B. ein physiologischer Zusammenhang besteht. Somit können funktionelle Auswertungen von z. B. den Herzkammern quantitativ verglichen werden, insbesondere mit mindestens einer vollständigen räumlich vergleichenden Auswertung.

Besonders bevorzugt enthalten nämlich die Datensätze mit funktionellen Werten räumlich aufgelöst zu allen oder fast allen Abschnitten der Kavität funktionelle Werte. Das heißt, beispielsweise die Bewegung der Herzkammer wird nicht nur punktuell in zwei oder drei Schnittbildern verglichen, sondern möglichst über den gesamten Raum bzw. dem Volumen der verglichenen Daten. Hierzu kann als einheitliches Format eine Projektion auf eine 2D Ebene wie zum Beispiel ein Polarplot gewählt werden. Sollten einige der Datensätze mit funktionellen Werten in einem anderen Format, zum Beispiel als Beutel-Datensatz oder in Form einer Wertetabelle vorliegen, so werden diese in ein einheitliches Datenformat z. B. auf einen Polarplot übertragen.

Falls notwendig, werden bei der Verrechnung weitere Berechnungen durchgeführt, zum Beispiel wird über den einem Sektor des Polarplots entsprechenden Abschnitt eines Beutel-Datensatzes gemittelt oder die Position jedes Punktes auf dem Beutel im Polarplot berechnet.

Besonders bevorzugt wurden die mindestens zwei Datensätze mit funktionellen Werten aus dynamischen Bildern des Herzens gewonnen, die bei unterschiedlichen Belastungszuständen des Herzens aufgenommen wurden. Eine typische Stressechountersuchung wird zum Beispiel in Ruhe, bei leichter körperlicher Belastung und bei starker körperlicher Belastung durchgeführt.

Alternativ kann die Erfindung auch dazu verwendet werden, Bilder des Herzens zu vergleichen, die mit unterschiedlichen medizinischen Bildgebungsverfahren bzw. -modalitäten aufgenommen wurden, zum Beispiel mit MRT und Ultraschall oder mit PET und Ultraschall. Das Verfahren kann auch für den Vergleich von Datensätzen mit funktionellen Werten verwendet werden, die mittels Computertomographie (CT), Magnetresonanztomographie, Katheteruntersuchungen (z.B. Potentialmessungen), PET oder SPECT aufgenommen wurden. Insofern kann das Verfahren auch auf nicht-bildgebende Verfahren wie die elektrophysiologische Herzkatheteruntersuchung (Potentialmessung am Herzen) verwendet werden.

Gemäß einer besonderen Ausführungsform können die Bilder der Kavität und/oder die Datensätze mit funktionellen Werten eine dynamische Zeitreihe aus dreidimensionalen Bilddatensätzen sein.

Falls die Datensätze bei der Umwandlung in das einheitliche Datenformat nicht auf die gleiche räumliche Auflösung (Pixelgröße) gebracht wurden, können die funktionellen Datensätze dennoch miteinander verrechnet werden. die funktionellen Datensätze werden dabei gegebenenfalls über- oder unterabgetastet, wenn sie mit unterschiedlicher räumlicher Auflösung vorliegen.
Die Darstellung der Ergebnis-Datensätze kann auf unterschiedliche Weise erfolgen. Zum Beispiel können alle ursprünglichen und verrechneten Polarplots oder Beutel-Datensäzte nebeneinander dargestellt werden. Gegebenenfalls können auch mehrere Polarplots übereinander (semitransparent) dargestellt werden, wobei zum Beispiel ein verschiebbares Fenster im oberen Polarplot vorgesehen ist, durch den der untere Polarplot sichtbar ist. Ferner ist es möglich, alle ursprünglichen und/oder verrechneten Werte auf einer Beuteldarstellung der Herzkammer zum Beispiel farbcodiert darzustellen.

Hierzu werden die zum Beispiel im Programm "4D LV-Analysis CRT" der Anmelderin berechneten und exportierten Werte der drei Stressecho-Datensätze geladen und miteinander verrechnet. Es werden dabei zum Beispiel die Differenzen der jeweiligen Werte zwischen den drei Datensätzen berechnet und wieder als Parameterkarte im Polarplot oder auf einem Beutel dargestellt.

Als eine Ausführungsform der Verrechnung können beliebige Boolsche Operationen (z. B. UND, ODER, UND NICHT) durchgeführt werden. Dies eignet sich insbesondere für funktionelle Werte, die auf binäre Werte (0 oder 1) reduziert werden können. Z.B. kann die Perfusion nach einem Infarkt in einem Abschnitt der Herzkammerwand zumindest bei Stress auf null absinken. Diese Information kann mit anderen funktionellen Werten, z.B. vorhandene Bewegung dieses Abschnitts, kombiniert werden, um ein dauerhaft geschädigtes Herzareal zu identifizieren. Die Verknüpfung mit Boolschen Operatorgen eignet sich auch zeitlich gesehen z.B. zur Visualisierung von Bewegungsmustern. Hierzu bildet man z.B. die symmetrische Differenz (Vereinigungsmenge ohne Schnittmenge) zweier Beutel, um Lageunterschiede im Raum darstellen zu können

Die Erfindung ist auch auf ein Computerprogrammprodukt gerichtet, welches auf einem Computer-lesbaren Medium gespeicherten Programmcode enthält, der eine Durchführung des oben beschriebenen Verfahrens bewirkt, wenn der Programmcode auf einem Computer ausgeführt wird.

Schließlich ist die Erfindung auch auf eine Vorrichtung zum Auswerten von Bildern einer Kavität, z. B. des Herzens, gerichtet, die mit einem medizinischen Bildgebungsverfahren aufgenommen wurden, welche insbesondere zur Ausführung des oben beschriebenen Verfahrens ausgelegt ist. Hierzu umfasst die Vorrichtung einen Datenspeicher, in dem Datensätze mit funktionellen Werten gespeichert sind, welche z. B. die Herzkammerwand-Aktivität einer Herzkammer beschreiben und aus den Bildern der Kavität berechnet wurden, wobei zumindest zwei Datensätze mit funktionellen Werten in jeweils einem beliebigen Datenformat, insbesondere in Form einer Wertetabelle, einer Projektion auf eine 2D Ebene oder einer Gitternetzstruktur im Datenspeicher vorgehalten werden; eine Recheneinheit, die dazu ausgelegt ist, den/die nicht in dem einheitlichen Datenformat bereitgestellten Datensätze mit funktionellen Werten in das einheitliche Datenformat umzuwandeln, und die im einheitlichen Datenformat vorliegenden Datensätze miteinander zu verrechnen (z. B. zu addieren oder zu subtrahieren), und um zumindest einen Ergebnis-Datensatz zu erzeugen; und einen Bildschirm, der zum Darstellen des Ergebnis-Datensatzes geeignet ist.

Die Erfindung wird nun an Hand von Ausführungsbeispielen mit Bezug auf die beiliegenden Zeichnungen näher erläutert.

### In den Zeichnungen zeigen:

- Fig. 1: einen Beutel-Datensatz;
- Fig. 2: einen Polarplot;
- Fig. 3: eine Wertetabelle;

- Fig. 4: ein Ausführungsbeispiel der Darstellung von ursprünglichen und Ergebnis-Datensätzen;
- Fig. 5: ein Ausführungsbeispiel einer erfindungsgemäßen Vorrichtung.

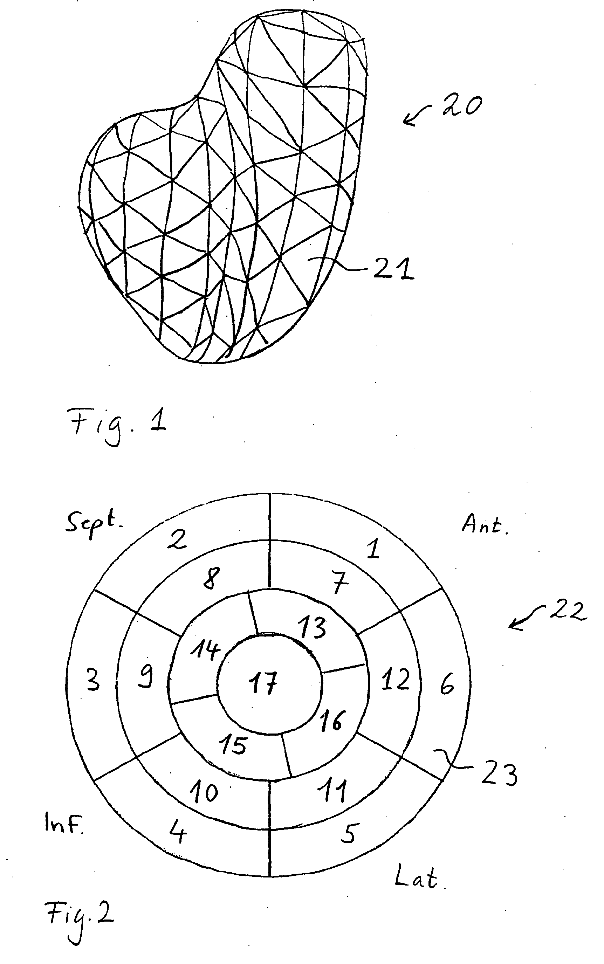

Fig. 1 illustriert das Beutelformat, in welchem die funktionellen Werte vorliegen können. Dieses Datenformat ist ausführlich in der EP 0 961 135 A beschrieben, deren Offenbarungsgehalt hiermit in diese Anmeldung aufgenommen wird. Es handelt sich hierbei um eine einem Beutel 20 ähnliche geometrische Gitternetzstruktur, welche durch einzelne Punkte oder, wie im dargestellten Beispiel, durch einzelne Dreiecke 21 repräsentiert ist. Ein derartiger Beutel 20 kann jeweils aus einem dreidimensionalen Bilddatensatz einer Kavität, zum Beispiel durch Ermitteln der Konturen zwischen Innenraum und Wand der Kavität ermittelt werden. Bei einer Herzkammer wird hierbei vorzugsweise die Grenzfläche zwischen Blut und Wand konturiert, wobei zusätzlich auch die Grenzfläche zwischen Wand und umgebenden Gewebe konturiert werden kann. Durch die Konturierung erhält man eine Oberfläche, die daraufhin parametrisiert wird. Für das Beutelformat werden dabei einzelne Punkte bzw. Dreiecke extrahiert, die einen Beutel aufspannen und in kartesischen Koordinaten (3D oder 4D) oder in sphärischen Koordinaten vorliegen. Ein dynamischer 3D-Datensatz kann also durch einen dynamischen, also zeitlich veränderlichen, Beutel-Datensatz repräsentiert werden. Zusätzlich können auch funktionelle Werte aus dem dynamischen Beutel-Datensatz ermittelt werden, zum Beispiel der Zeitpunkt der Kontraktion oder dergleichen, und als farbige Einfärbung auf dem Beutel 20 dargestellt werden.

Eine alternative Darstellungsform ist der Polarplot 22, der in Fig. 2 dargestellt ist. Hierbei handelt es sich um eine nach bestimmten Regeln projizierte Wiedergabe z. B. der Herzkammerwand auf eine 2D Ebene, die in mehrere Sektoren 23 eingeteilt ist. Der Sektor 17 liegt beispielsweise an der Beutelspitze (z. B. am Apex), der Sektor 1 am anterioren Ende der Kammer. Auch ein Polarplot kann aus einem 3D Bild des Herzens ermittelt werden, indem die Grenzfläche zwischen Blut und Wand konturiert wird. Die Position des Apex wird ermittelt, und die ermittelte Oberfläche wird parametrisiert, indem für jeden gewünschten Punkt der Oberfläche die beiden Winkel in Bezug auf den Apex (Ursprung des Koordinatensystems) berechnet werden. Dies erlaubt bereits die Darstellung als Polarplot. Ggf. wird zusätzlich der Abstand zum Ursprung ermittelt. Ein Polarplot kann eine beliebig hohe oder niedrige räumliche Auflösung aufweisen.

Der Polarplot kann zur Darstellung verschiedener funktioneller Werte verwendet werden, zum Beispiel der zeitlichen Verzögerung der Kontraktion oder aber der maximalen volumenänderung bei jedem Sektor. Dazu werden die einzelnen Werte wiederum farbcodiert und als Landkarte auf dem Polarplot eingetragen.

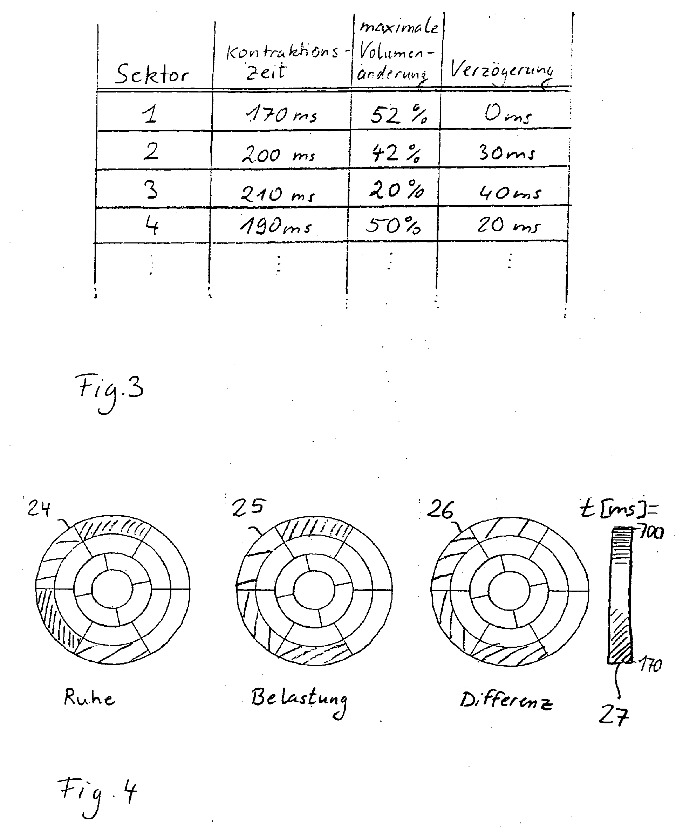

Eine weitere Alternative zur Darstellung funktioneller Werte zur Herzkammerwand-Aktivität ist in Fig. 3 dargestellt, nämlich eine Wertetabelle. Auch diese Wertetabelle ist nach räumlich definierten Bereichen (auflösungsabhängig bis auf Punktniveau) der Herzkammerwand geordnet. Der Vorteil einer Wertetabelle liegt darin, dass verschiedene funktionelle Werte, hier zum Beispiel die Kontraktionszeit, die maximale Volumenänderung und die Verzögerung in ms, gleichzeitig dargestellt werden können. Nachteilig ist, dass keine Visualisierung erfolgt und die Ergebnisse daher nicht mit einem Blick erfasst werden können.

Gemäß einem Ausführungsbeispiel des erfindungsgemäßen Verfahrens ist es nun möglich, die in den drei unterschiedlichen Formaten vorliegenden Datensätze mit funktionellen Werten jeweils in ein einheitliches Format umzuwandeln, zum Beispiel in einen Polarplot, und diese dann untereinander zu vergleichen. Im dargestellten Beispiel wird zum Beispiel aus der Wertetabelle der Fig. 3 an Hand der Nummern der Sektoren ein Polarplot gemäß Fig. 2 ermittelt, welcher allerdings eine geringere Auflösung hat als andere Polarplots. Bei einem Polarplot gemäß Fig. 2 muss nicht jeder Pixel in einem Sektor den gleichen Wert haben. Darüber hinaus können auch die auf einem Beutel visualisierten funktionellen Werte in einen Polarplot umgewandelt werden. Auf diese Weise ist ein direkter Vergleich verschiedener Arten von Daten möglich.

In Fig. 4 ist zum Beispiel eine entsprechende Darstellung der Ergebnis-Datensätze gezeigt. Hier bezeichnet 24 einen Polarplot des linken Ventrikels im Ruhezustand. Im Polarplot sind beispielsweise die Kontraktionszeiten in ms farblich dargestellt, gemäß der Farbskala 27. Der Polarplot 25 stellt den gleichen funktionellen Wert im Belastungszustand dar. Polarplot 26 zeigt nun den Ergebnis-Datensatz, in diesem Fall eine Differenz zwischen den Datensätzen der Polarplots 24 und 25. Für den Polarplot 26 kann auch eine andere Farbskala gelten als für die beiden anderen Plots. Erfolgt die Darstellung auf einem Monitor mit Computeranschluss, kann der Benutzer auch zum Beispiel durch Überfahren des Polarplots 26 mit einer Maus den an jeder Stelle ermittelten Vergleichswert ablesen.

Die auf diese Weise erhaltenen Ergebnis-Datensätze sind im Gegensatz zur konventionellen Auswertung objektiv und nicht abhängig vom Untersucher. Daher sind sie auch in Verlaufsuntersuchungen verwendbar.

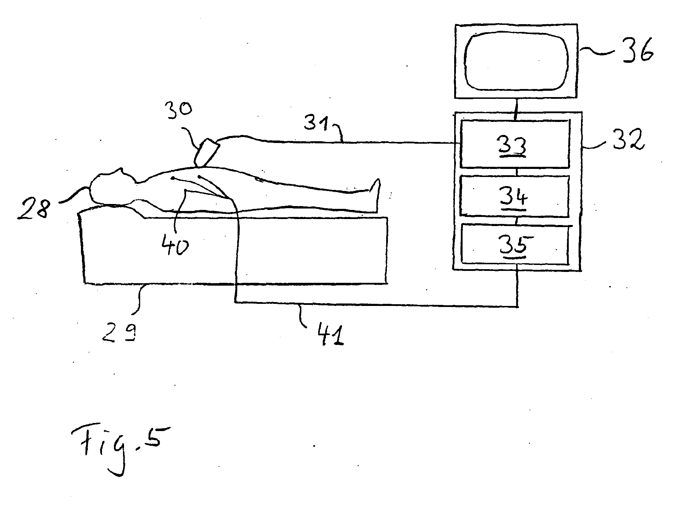

In Fig. 5 ist schließlich ein Ausführungsbeispiel einer erfindungsgemäßen Vorrichtung gezeigt. Darin ist ein Patient 28 auf einer Liege 29 positioniert. Ultraschallaufnahmen z. B. des Herzens werden mit dem Ultraschallkopf 30 aufgenommen und über das Kabel 31 an die Auswerteeinheit 32 übermittelt. Diese umfasst eine Recheneinheit 33 und einen Datenspeicher 34. Ferner kann optional auch ein EKG-Gerät 35 vorgesehen sein. Dieses ist über ein Kabel 41 mit mehreren Elektroden 40 verbunden, mit denen während der Ultraschallaufnahmen ein EKG aufgenommen werden kann. Dieses erleichtert die Zuordnung der aufgenommenen Bilder zu bestimmten Herzphasen.

Das erfindungsgemäße Verfahren kann zum Beispiel von der Recheneinheit 33 direkt nach Aufnahme der dynamischen Bilder durchgeführt werden. Zur Darstellung der Ergebnis-Datensätze ist ein Bildschirm 36 vorgesehen.

## Patentansprüche

1. Verfahren zum Auswerten von Bildern einer Kavität im menschlichen oder tierischen Körper, die mit einem oder mehreren verschieden medizinischen Bildgebungsverfahren aufgenommen wurden,
bei welchem Datensätze mit funktionellen Werten verwendet werden, welche aus den Bildern der Kavität berechnet wurden, wobei das Verfahren **dadurch gekennzeichnet ist, daß** es die folgenden Schritte aufweist:
- Bereitstellen von mindestens zwei Datensätzen mit funktionellen Werten in unterschiedlichen Datenformaten, insbesondere in Form einer Wertetabelle, einer Projektion auf eine 2D Ebene (22) oder einer Gitternetzstruktur (20);
- Bereitstellen eines einheitlichen Datenformates;
- Umwandeln des/der nicht in dem einheitlichen Datenformat bereitgestellten Datensätze mit funktionellen Werten in das einheitliche Datenformat (22);
- Verrechnen der im einheitlichen Format vorliegenden Datensätze (24, 25) miteinander zur Erzeugung von zumindest einem Ergebnis-Datensatz (26);
- Darstellen des Ergebnis-Datensatzes.

2. Verfahren nach Anspruch 1, bei welchem die Kavität eine Herzkammer ist.

3. Verfahren nach Anspruch 2, bei welchem die Datensätze mit funktionellen Werten jeweils einen der folgenden funktionellen Werte für mehrere räumliche Abschnitte der Herzkammer repräsentieren: die Bewegung der Herzkammerwand, die relative Auslenkung der Herzkammerwand, die zeitliche Verzögerung der maximalen Auslenkung der Herzkammerwand, die Volumenänderung eines Abschnitts der Herzkammer, die Wanddicke, die Perfusion, den Strain, die Strain Rate oder die elektrischen Potentiale der Herzkammerwand.

4. Verfahren nach einem der vorhergehenden Ansprüche, bei welchem das einheitliche Datenformat ein Polarplot (22) ist.

5. Verfahren nach einem der vorhergehenden Ansprüche, bei welchem die im einheitlichen Format vorliegenden Datensätze (24, 25) beim Verrechnen miteinander addiert, voneinander subtrahiert oder durch Boolsche Operatoren miteinander kombiniert werden.

6. Verfahren nach einem der vorhergehenden Ansprüche, bei welchem die Bilder der Kavität zumindest teilweise dynamische Bilder sind.

7. Verfahren nach Anspruch 6, bei welchem die Bilder der Kavität und/oder die Datensätze mit funktionellen Werten eine Zeitreihe aus dreidimensionalen Bilddatensätzen sind.

8. Verfahren nach einem der Ansprüche 2 bis 7, bei welchem die Bilder der Herzkammer 4D-Stressecho-Bilddatensätze sind.

9. Verfahren nach einem der vorhergehenden Ansprüche, bei welchem die Datensätze mit funktionellen Werten räumlich aufgelöst zu allen oder fast allen Abschnitten der Kavität funktionelle Werte enthalten.

10. Verfahren nach einem der Ansprüche 2 bis 9, bei welchem die mindestens zwei Datensätze mit funktionellen Werten aus Bildern des Herzens gewonnen wurden, die bei unterschiedlichen Belastungszuständen des Herzens aufgenommen wurden.

11. Verfahren nach einem der vorhergehenden Ansprüche, bei welchem die mindestens zwei Datensätze mit funktionellen Werten aus Bildern der Kavität gewonnen wurden, die mit unterschiedlichen medizinischen Bildgebungsverfahren aufgenommen wurden.

12. Verfahren nach einem der vorhergehenden Ansprüche, bei welchem die Bilder der Kavität mittels Ultraschall, Röntgen, Computertomographie, Magnetresonanztomographie, elektrophysiologischer Katheteruntersuchung, Positronen-Emissionstomographie oder SPECT aufgenommen wurden.

13. Verfahren nach einem der vorhergehenden Ansprüche, bei welchem die mindestens zwei Datensätze aus funktionellen Werten zwar in ein einheitliches Format, jedoch mit unterschiedlicher räumlicher Auflösung, umgewandelt werden, wobei beim Verrechnen (z. B. Addieren oder Subtrahieren) der im einheitlichen Format vorliegenden Datensätze der/die Datensätze mit geringerer räumlicher Auflösung überabgetastet und/oder der/die Datensätze mit höherer räumlicher Auflösung unterabgetastet werden.

14. Verfahren nach einem der vorhergehenden Ansprüche, bei welchem die Ergebnis-Datensätze (26) in einer Projektion auf eine 2D Ebene, insbesondere in einem Polarplot (22), dargestellt werden.

15. Computerprogrammprodukt, welches auf einem computerlesbaren Medium gespeicherten Programmcode enthält, der eine Durchführung des Verfahrens nach einem der vorhergehenden Ansprüche bewirkt, wenn der Programmcode auf einem Computer ausgeführt wird.

16. Vorrichtung zum Auswerten von Bildern einer Kavität im menschlichen oder tierischen Körper, die mit einem oder mehreren medizinischen Bildgebungsverfahren aufgenommen wurden, wobei die Vorrichtung umfasst:
- einen Datenspeicher (34), in dem Datensätze mit funktionellen Werten gespeichert sind, welche aus den Bildern der Kavität berechnet wurden, **dadurch gekennzeichnet, daß** mindestens zwei Datensätze mit funktionellen Werten in jeweils unterschiedlichen Datenformaten, insbesondere in Form einer Wertetabelle, einer Projektion auf eine 2D Ebene (22) oder einer Gitternetzstruktur (20), im Datenspeicher vorgehalten werden;
- einen Datenspeicher (34), in dem ein einheitliches Datenformat gespeichert ist;
- eine Recheneinheit (33), die dazu ausgelegt ist, den/die nicht in dem einheitlichen Datenformat bereitgestellten Datensätze mit funktionellen Werten in das einheitliche Format umzuwandeln, und die im einheitlichen Format vorliegenden Datensätze miteinander zu verrechnen, insbesondere zu addieren oder zu subtrahieren, um zumindest einen Ergebnis-Datensatz (26) zu erzeugen;
- einen Bildschirm (36), der zum Darstellen des Ergebnis-Datensatzes geeignet ist.

17. Vorrichtung nach Anspruch 16, welche zum Ausführen des Verfahrens nach einem der Ansprüche 1 bis 14 eingerichtet ist.

## Claims

1. Method of evaluating images of a cavity in the human or an animal body, which have been acquired with one or several different medical imaging methods,
wherein data sets with functional values are used, which have been computed from the images of the cavity, the method being **characterized in that** it comprises the following steps:
- providing at least two data sets with functional values in different data formats, in particular in the form of a data table, a projection on to a 2D plane (22), or a grid network structure (20);
- providing a uniform data format;
- conversion of the data set(s) with functional values not made available in the uniform data format into the uniform data format (22);
- computation of the data sets (24, 25) present in the uniform data format with one another to generate at least one result data set (26);
- representation of the result data set.

2. Method according to claim 1, wherein the cavity is a heart chamber.

3. Method according to claim 2, wherein the data sets with functional values each represent one of the following functional values for plural spatial sections of the heart chamber: the movement of the heart chamber wall, the relative displacement of the heart chamber wall, the time delay of the maximum displacement of the heart chamber wall, the volume change of a section of the heart chamber, the wall thickness, the perfusion, the strain, the strain rate or the electrical potentials of the heart chamber wall.

4. Method according to one of the preceding claims, in which the uniform data format is a polar plot (22).

5. Method according to one of the preceding claims,
wherein the computation of the data sets (24, 25) present in the uniform format comprises adding together, subtracting from one another or combining the data sets (24, 25) with one another by Boolean operators.

6. Method according to one of the preceding claims,
wherein the images of the cavity are at least in part dynamic images.

7. Method according to claim 6, wherein the images of the cavity and/or the data sets with functional values are a time sequence composed of three-dimensional image data sets.

8. Method according to one of claims 2 to 7, wherein the images of the heart chamber are 4D stress echo image data sets.

9. Method according to one of the preceding claims,
wherein the data sets with functional values contain functional values resolved spatially to all or almost all sections of the cavity.

10. Method according to one of claims 2 to 9, wherein the at least two data sets with functional values have been obtained from images of the heart, which have been acquired in different states of stress of the heart.

11. Method according to one of the preceding claims,
wherein the at least two data sets with functional values have been obtained from images of the cavity which have been acquired with different medical imaging methods.

12. Method according to one of the preceding claims,
wherein the images of the cavity have been acquired by means of ultrasound, X-ray, Computer Tomography, Magnetic Resonance Imaging, electrophysiological catheter test, Positron Emission Tomography or SPECT.

13. Method according to one of the preceding claims,
wherein the at least two data sets composed of functional values are converted into a uniform format, but with different spatial resolution, wherein during the computation (e.g. adding or subtracting) of the data sets present in the uniform format, the data set(s) with lower spatial resolution are over-sampled and/or the data set(s) with a higher spatial resolution are under-sampled.

14. Method according to one of the preceding claims,
wherein the result data sets (26) are represented in a projection on to a 2D plane, in particular in a polar plot (22) .

15. Computer program product, which contains program code stored on a computer-readable medium, which performs the method according to one of the preceding claims if the program code is executed on a computer.

16. Device for evaluating images of a cavity in the human or an animal body, which have been acquired with one or several medical imaging methods, wherein the device comprises:
- a data store (34), in which data sets with functional values are stored, which have been computed from the images of the cavity, **characterized in that** at least two data sets with functional values have been provided in respectively different data formats, in particular in the form of a data table, a projection on to a 2D plane (22) or a grid network structure (20) in the data store;
- a data store (34), in which a uniform data format is stored;
- a computing unit (33), which is adapted to convert the data sets with functional values not provided in a uniform data format into the uniform format, and to compute the data sets present in the uniform format with one another, in particular to add them or subtract them, in order to produce at least one result data set (26);
- a screen (36) suitable for displaying the result data set.

17. Device according to claim 16, which is arranged to carry out the method according to one of claims 1 to 14.

## Revendications

1. Procédé pour l'évaluation d'images d'une cavité dans un corps humain ou animal, qui ont été prises par un ou plusieurs procédés différents d'imagerie médicale,
dans lequel on utilise des lots de données avec des valeurs fonctionnelles qui ont été calculées à partir des images de la cavité,
dans lequel le procédé est **caractérisé en ce qu'**il comprend les étapes suivantes :
- préparation d'au moins deux lots de données avec des valeurs fonctionnelles dans des formats de données différents, en particulier sous la forme d'une table de valeurs, d'une projection sur un plan bidimensionnel (22) ou d'une structure en réseau maillé (20) ;
- préparation d'un format de données unitaire ;
- conversion des lots de données avec des valeurs fonctionnelles qui n'ont pas été préparées dans le format de données unitaire, vers le format de données unitaire (22) ;
- compensation des lots de données (24, 25) qui se présentent dans le format unitaire les uns avec les autres pour générer au moins un lot de données résultant (26) ;
- présentation du lot de données résultant.

2. Procédé selon la revendication 1, dans lequel la cavité est une chambre cardiaque.

3. Procédé selon la revendication 2, dans lequel les lots de données avec des valeurs fonctionnelles représentent respectivement l'une des valeurs fonctionnelles suivantes pour plusieurs tronçons locaux de la chambre cardiaque : le déplacement de la paroi de la chambre cardiaque, la déflexion relative de la paroi de la chambre cardiaque, le retard temporel de la déviation maximale de la paroi de la chambre cardiaque, la variation de volume d'un tronçon de la chambre cardiaque, l'épaisseur de paroi, la perfusion, la valeur "Strain", la valeur "Strain Rate", ou les potentiels électriques de la paroi de la chambre cardiaque.

4. Procédé selon l'une des revendications précédentes, dans lequel le format de données unitaire est un diagramme polaire (22).

5. Procédé selon l'une des revendications précédentes, dans lequel les lots de données (24, 25) qui se présentent sous le format unitaire sont ajoutés les uns avec les autres, soustraits les uns des autres, ou combinés les uns avec les autres par des opérateurs booléens lors de la compensation.

6. Procédé selon l'une des revendications précédentes, dans lequel les images de la cavité sont des images au moins partiellement dynamiques.

7. Procédé selon la revendication 6, dans lequel les images de la cavité et/ou les lots de données avec valeurs fonctionnelles sont une série temporelle de lots de données images tridimensionnelles.

8. Procédé selon l'une des revendications 2 à 7, dans lequel les images de la chambre cardiaque sont des lots de données image dits "4D-stressecho" (écho sous stress en 4D).

9. Procédé selon l'une des revendications précédentes, dans lequel les lots de données avec valeurs fonctionnelles contiennent des valeurs fonctionnelles à résolution spatiale pour tous les tronçons de la cavité ou presque tous.

10. Procédé selon l'une des revendications 2 à 9, dans lequel lesdits au moins deux lots de données avec valeurs fonctionnelles ont été récupérés à partir d'images du coeur qui ont été prises dans des états de charges différents du coeur.

11. Procédé selon l'une des revendications précédentes, dans lequel lesdits au moins deux lots de données avec valeurs fonctionnelles ont été récupérés à partir d'images de la cavité qui ont été prises avec des procédés différents d'imagerie médicale.

12. Procédé selon l'une des revendications précédentes, dans lequel les images de la cavité ont été prises avec des ultrasons, des rayons X, par tomographie à l'ordinateur, par tomographie à résonance magnétique, par analyse au cathéter électrophysiologique, par tomographie par émission de positrons, ou par un procédé dit "SPECT".

13. Procédé selon l'une des revendications précédentes, dans lequel lesdits au moins deux lots de données avec des valeurs fonctionelles sont convertis, certes dans un format unitaire mais avec une résolution spatiale différente, et lors de la compensation (par exemple addition ou soustraction) des lots de données qui se présentent sous un format unitaire, on procède à un sur-échantillonnage du/des lots de données avec résolution spatiale plus faible et/ou à un sous-échantillonnage du/des lots de données avec résolution spatiale plus élevée.

14. Procédé selon l'une des revendications précédentes, dans lequel les lots de données résultants (26) sont représentés dans une projection sur un plan bidimensionnel, en particulier en un diagramme polaire (22).

15. Produit de programme d'ordinateur, qui contient un code de programme mémorisé sur un support lisible à l'ordinateur, qui entraîne une mise en oeuvre du procédé selon l'une des revendications précédentes quand le code de programme est exécuté sur un ordinateur.

16. Appareil pour évaluer des images d'une cavité dans des corps humains ou animaux, qui ont été prises avec un ou plusieurs procédés d'imagerie médicale, dans lequel l'appareil comprend :
- une mémoire de données (34) dans laquelle sont mémorisés des lots de données avec valeurs fonctionnelles, qui ont été calculés à partir des images de la cavité,
**caractérisé en ce que**
au moins deux lots de données avec valeurs fonctionnelles dans des formats de données respectivement différents, en particulier sous la forme d'une table de valeurs, d'une projection sur un plan bidimensionnel (22) ou d'une structure à réseau maillé (20), sont conservés dans la mémoire de données ;
- une mémoire de données (34) dans laquelle un format de données unitaires est mémorisé ;
- une unité de calcul (33), conçue pour convertir le/les lots de données avec valeurs fonctionnelles qui ne sont pas préparées dans le format unitaire, vers le format unitaire, et de compenser les uns avec les autres les lots de données qui se présentent dans le format unitaire, en particulier les additionner ou les soustraire, pour engendrer au moins un lot de données résultant (26) ;
- un écran (36), approprié pour présenter le lot de données résultant.

17. Appareil selon la revendication 16, conçu pour mettre en oeuvre le procédé selon l'une des revendications 1 à 14.
